# EUROPÄISCHE PATENTSCHRIFT

(11) **EP 2 996 756 B1**
(45) Veröffentlichungstag und Bekanntmachung des Hinweises auf die Patenterteilung: **16.12.2020**
(21) Anmeldenummer: 14738365.7
(22) Anmeldetag: 15.05.2014
(51) Int. Cl.: A61M 35/00

(54) **HAUTAPPLIKATOR ZUR APPLIKATION VON LEICHTFLÜCHTIGEN SUBSTANZEN**
SKIN APPLICATOR FOR APPLYING HIGHLY VOLATILE SUBSTANCES
APPLICATEUR CUTANÉ POUR APPLIQUER DES SUBSTANCES VOLATILES

(30) Priorität: 15.05.2013 DE 102013008237
(43) Veröffentlichungstag der Anmeldung: 23.03.2016
(73) Patentinhaber: Solvamed GmbH, 60318 Frankfurt (DE)
(72) Erfinder: MARGRAF, Stefan, 60318 Frankfurt (DE)
(74) Vertreter: Patentanwälte Bauer Vorberg Kayser
(86) Internationale Anmeldenummer: PCT/DE2014/200221
(87) Internationale Veröffentlichungsnummer: WO 2014/183764

(56) Entgegenhaltungen:
- WO-A1-01/91853
- WO-A1-02/43611
- WO-A2-2008/077114
- WO-A2-2008/077114
- DE-A1- 2 055 159
- US-A- 5 516 505
- US-A1- 2010 094 249

## Beschreibung

Die vorliegende Erfindung betrifft einen Hautapplikator zur Applikation von leichtflüchtigen Substanzen, für Chloroform oder Dietylether oder wässrige Lösungen von Diethylether auf die Haut gemäß dem Patentanspruch 1.

Seit mehr als 60 Jahren ist die inaktivierende Wirkung von Dietylether (im folgenden auch Ether) unter anderem gegen Viren der Herpes Gruppe bekannt (Albert S. Kaplan Department of Bacteriology, University of Illinois, Urbana, Illinois, USA, Virology Volume 4, Issue 3, December 1957, Pages 435-457, A study of the herpes simplex virus-rabbit kidney cell system by the plaque technique).

Ether zerstört offenbar die Lipid-Hülle von Viren, auf die Haut aufgetragen kommt es darüber hinaus zur Kühlung, Schmerzstillung und Austrocknung. Ether ist vorteilhafterweise, im Gegensatz zum Beispiel zu Ethanol, in der Lage in die Haut in wirksamer Menge einzudringen ohne diese nennenswert zu schädigen. Viele Millionen Ethernarkosen der vergangenen Jahrzehnte haben hierbei die Sicherheit von Etheranwendungen bewiesen. Mischungen von Ether mit (Acetyl-)Salicylsäure (Bareggi SR, Pirola R, De Benedittis G. Department of Pharmacology, University of Milan, Italy. Eur J Clin Pharmacol. 1998 May;54(3):231-5, Skin and plasma levels of acetylsalicylic acid: a comparison between topical aspirin/diethyl ether mixture and oral aspirin in acute herpes zoster and postherpetic neuralgia) wurden in der Literatur als vorteilhaft zur Therapie von Herpes beschrieben.

Auch der leicht flüchtige Stoff Chloroform inaktiviert Herpes Viren (Taylor CA, Hendley JO, Greer KE, Gwaltney JM Jr. Arch Dermatol. 1977 Nov;113(11):1550-2. Topical treatment of herpes labialis with chloroform).Die virusinaktivierende und andere Wirkung von Ether auf Haut bei Vorliegen einer Herpesinfektion wurde in kleineren Studien mit zum Teil zweifelhafter Qualität untersucht. Es gibt dazu positive (Farrell RG, Nesland RS. . JACEP. 1977 Aug;6(8):372-3, Topical ethyl ether therapy of herpes simplex lesions;. H. D. Mintun, MD . JAMA. 1978;239(9):831. Application of Diethyl Ether to Herpes Simplex Lesions) als auch negative Berichte (Guinan ME, MacCalman J, Kern ER, Overall JC Jr, Spruance SL. JAMA. 1980 Mar 14;243(10):1059-61, Topical ether and herpes simplex labialis). Exemplarisch eingehend auf die (negative) Untersuchung von Guinan wurden jedoch mehrere Fehler gemacht:
- Positive Wirkung einer Behandlung wurde empfunden von 77% der Probanden mit "Placebo" und 75% der Probanden mit Ether
- Als "Placebo" wurde eine Lösung von 5%Ether in Wasser verwendet in der irrigen Annahme, diese sei wirkungslos
- Die Läsionen der Ether-Gruppe waren fast dreifach größer im Vergleich zur "Placebo" Gruppe (55/152mm²)
- Die Probandenzahl mit n=51 war zu klein

Ether löst sich maximal zu 7% in Wasser, und entfaltet hier dennoch einen hohen Dampfdruck. Bei Applikation von Ether auf Haut würde demnach auch nur maximal 7% des Ethers in der Haut/Herpesbläschen in Lösung gehen, da diese größtenteils aus Wasser besteht. Damit war die Placebovariante wirkungsvoll, welches nicht nur die positive Wirkung erklärt sondern auch, dass das gemessene Ausmaß der positiven Wirkung viel größer war als jene bei großen Studien mit Acyclovir.

Ether wurde in den bisherigen Studien meist mit kleinen Wattebäuschen aus Viskose/Baumwolle, welche an ein Stäbchen befestigt sein können (Ohrenstäbchen), aufgetragen. Durch die niedrige Siedetemperatur von Ether (34,6°C) verbunden mit dem hohen Dampfdruck (586hPa gemessen bei 20°C) ist die beschriebene Applikationsform unvorteilhaft. Der im Wattebausch enthaltende Ether kann durch die Haut bis auf Siedetemperatur erwärmt werden und verdampft. Auch die große, nicht die Haut kontaktierende Oberfläche der Watte, führt zu einer schnellen Verdampfung des Ethers in die umgebende Luft innerhalb von Sekunden. Sollte eine Zone des Gesichts so behandelt werden, so wird viel von dem verdampften Ether eingeatmet. Das ist unerwünscht und führt frühzeitig zum Abbruch der Behandlung. Die Haut wird dementsprechend nur sehr wenig mit Ether kontaktiert. Eine optimale Wirkungsentfaltung ist hierbei nicht gegeben.

Da es keine geeignete Applikationsform auf Haut für flüssige Stoffe oder Gemische mit hohem Dampfdruck, definiert als größer als 57hPa, weiter bevorzugt größer als 100hPa (jeweils gemessen bei 20°C und 1013mbar), gibt, ist kein Produkt am Markt, um beispielsweise Ether zur Anwendung zu bringen.

In diesem Zusammenhang sind leichtflüchtige Flüssigkeiten Stoffe oder Gemische, welche bei einer Temperatur von 20°C eine Flüssigphase haben sowie mindestens den oben erwähnten Dampfdruck von 57hPa, bevorzugt mindestens 100 hPa, weiter bevorzugt mindestens 200 hPa aufweisen. Die Flüssigkeiten können auch gelöste Gase enthalten, welche als Reinstoff bei obigen Bedingungen gasförmig sind.

Siehe auch WO2008/077114, US2010/094249 und WO01/91853.

Um für dieses Problem eine Lösung zu generieren ist hier folgende Erfindung gemacht worden:
Ein auf einen Flüssigkeitsbehälter angebrachter Deckel enthält eine integrierte Dosiersprüheinrichtung charakterisiert dadurch, dass bei einmaligem (Pump-) Sprühstoß eine Menge von 25-600µl Flüssigkeit (z. B. Ether oder Mischungen mit Ether) in eine Kammer von 0,1-8cm³ gesprüht wird und die Kammer eine Öffnung hat, welche auf ein Hautareal aufgedrückt wird in einer Weise, dass die Flüssigkeit und ihre Gase die Haut benetzen und kontaktieren, und dadurch die Flüssigkeit und ihre Gase mit der Haut über einen längeren Zeitraum interagieren können ohne vorzeitig zu verdunsten. Die Fläche der Haut, welche den Raum abschließt und mit dem Ether in Kontakt ist, beträgt bevorzugt etwa 4-250mm², auch bevorzugt 10-100mm². In dem Raum kann auch etwas Watte, Vlies oder offenporiger Schaum sein um spontanes Ablaufen der Flüssigkeit bei Undichtigkeit der Kammer-Haut Kontaktstelle zu vermeiden. Auf diese Weise wird wenig der Flüssigkeit oder ihrer Gase während der Behandlung an die Außenluft abgegeben und die Behandlung kann beliebig lange durchgeführt werden, zum Beispiel 1-10min. Wenn die Flüssigkeit, zum Beispiel Ether, von der Haut resorbiert worden ist, kann durch weitere Sprühstöße erneut Flüssigkeit in die Kammer abgegeben werden. Nach Beendigung der Maßnahme wird die Öffnung von der Haut abgehoben, wobei restliche, in der Kammer befindliche Flüssigkeit verdampft. Durch diese Erfindung ist es nun möglich eine leichtflüchtige Substanz für einen beliebigen Zeitraum, bevorzugt 1-10min, mit der Oberfläche eines Hautareals zuverlässig als Flüssig- oder Gasphase interagieren zu lassen. Außer Ether haben auch andere Stoffe eine desinfizierende Wirkung und einen hohen Dampfdruck, wie zum Beispiel Chloroform, Ethanol, Methanol, Isopropylalkohol, Flurane, usw..

Diethyl ether kann auch gelöst sein in Wasser zu 50-75g Diethyl ether pro Liter Wasser.

Die Dosiersprüheinrichtung kann manuell mit den Fingern ähnlich einer Nasentropfensprühflasche oder durch Druck auf die Haut ausgelöst werden. Auch bei Druck auf die Haut kann ein Ventil, welches durch Federkraft in einer Verschlussposition ist, durch überwinden der Federkraft in eine geöffnete Position verbracht werden ähnlich einem Lackstift. Bevorzugt werden jene Dosiersprüheinrichtungen, welche bei Betätigung eine begrenzte Menge freigeben. Solche Dosiersprüheinrichtungen sind dem Fachmann bekannt. Die Dosiersprüheinrichtung verschließt das Reservoir, welches ein Fläschchen oder eine Dose sein kann. Das Reservoir der Dosiersprüheinrichtung kann unter dem Dampfdruck der flüchtigen Substanz stehen oder zusätzlich ein Treibgas beinhalten.

Weitere Beispiele beziehen sich auf eine medizinische Vorrichtung zur Applikation von leicht flüchtigen Flüssigkeiten mit einem Dampfdruck von größer als 57hPa, bevorzugt mindestens 100 hPa, weiter bevorzugt mindestens 200 hPa bei 20C°gemessen, auf Haut dadurch gekennzeichnet, dass eine Menge Flüssigkeit in eine Kammer mit einer Öffnung bzw. Austrittsfläche, die auf die zu behandelnde Haut dichtend aufgesetzt und dadurch verschlossen ist, eingespritzt wird und dass das die Substanz kontaktierende Hautareal bzw. die Austrittsfläche eine Fläche von 4-250mm² aufweist.

Die Erfindung, wie in Anspruch 1 beansprucht, umfasst einen Hautapplikator für leichtflüchtige Flüssigkeiten mit einem Vorratsbehälter, einer mit dem Vorratsbehälter gekoppelten Dosiervorrichtung zur Förderung eines vorbestimmbaren Flüssigkeitsvolumens, sowie einem Applikatoraufsatz mit einer Applikationskammer, wobei die Applikationskammer einerseits mit der Dosiervorrichtung verbunden ist und andererseits eine von einer umlaufenden Kante begrenzte Applikationsöffnung zum Aufsetzen auf die Haut aufweist.

Leichtflüchtige Flüssigkeiten oder Substanzen sind solche mit einem Dampfdruck von mehr als 57hPa gemessen bei 20°C, vorzugsweise solche mit einem Dampfdruck von 100 hPa oder mehr, z.B. 150, 200 oder 250 hPa Geeignete Flüssigkeiten bzw. Substanzen sind an anderer Stelle beschrieben.

Die Dosiervorrichtung kann entweder eine Pumpdosiervorrichtung sein, welche das flüssige Medium durch manuellen Druck auf den Auslöser pumpt und dosiert, oder im Fall von Treibgasanwendung eine Dosiereinrichtung, die ohne die Notwendigkeit des Förderns der Flüssigkeit durch Pumpen arbeitet. Der Vorratsbehälter kann jede geeignete Form aufweisen, bevorzugt ist er zylindrisch, kann jedoch auch elliptisch oder eckig sein. Er weist bevorzugt die Dimensionen einer handelsüblichen, für Arzneimittel geeigneten Sprühdose auf.

Der Hautapplikator bzw. die Vorrichtung ist so ausgestaltet, dass er bis auf eine/die Applikationsöffnung im Wesentlichen geschlossen ist. Mit anderen Worten weist der Hautapplikator bzw. die Vorrichtung keine weiteren Öffnungen zur Umgebung auf.

Bevorzugt bildet die Applikationskammer bzw. der Aufsatz nach Aufsetzen des Hautapplikators bzw. der Vorrichtung auf ein Hautareal auf ein Hautareal ein Volumen von 0,1-8cm³, wie z.B. zwischen 0,5 und 5 cm³, z.B. 0,8 1, 2, 3, 4, 5, 6 oder 7 cm³ oder jeglicher dazwischen liegender Wert.

In ein solches Volumen der Applikationskammer kann dann eine Menge von 25-600µl einer leicht flüchtigen Flüssigkeit oder Substanz, z.B. 60, 100, 200, 250, 300, 400 oder 500 µl, mittels ein- oder mehrmaliger Auslösung einer Dosiervorrichtung in die Applikationskammer eingebracht werden. Die leicht flüchtige Flüssigkeit bzw. Substanz kann auf diese Weise mit der Haut interagieren.

Das Volumen an freigesetzter Flüssigkeit/Substanz kann demnach zwischen 25-600µl betragen, z.B. 50, 100, 150, 200, 250, 300, 400 oder 500 µl.

Die leichtflüchtige Flüssigkeit bzw. Substanz ist Diethylether oder Chloroform oder Gemische mit Diethylether. Die leichtflüchtige Flüssigkeit ist im Vorratsbehälter des Hautapplikators bzw. der Vorrichtung enthalten.

Bei Mischungen von Diethylether und anderen Substanzen (z.B. Chloroform, Ethanol, Methanol, Isopropylalkohol, Flurane, Wasser) sind alle Mischungen mit einem Dampfdruck von größer als 57hPa, weiter bevorzugt größer als 100hPa (jeweils gemessen bei 20°C und 1013 mbar) bevorzugt. Besonders bevorzugt sind Gemische von Diethylether mit Chloroform.

In der Öffnung bzw. Applikationskammer des Hautapplikators bzw. der Vorrichtung kann ein gegenüber der leichtflüchtigen Flüssigkeit wie z.B. Diethylether oder Chloroform stabiles Material wie z.B. Watte, Vlies oder ein offenporiger Schaum angeordnet sein. Dies kann helfen, um spontanes Ablaufen der Flüssigkeit bei Undichtigkeit der Kammer-Haut Kontaktstelle zu vermeiden. Die Dichte des porösen Materials beträgt bevorzugt unter 0,5g/cm³, z.B. weniger als 0,4 g/cm³, 0,3 g/cm³ oder 0,2 g/cm³. Ein Vlies kann z.B. Glasfasern, Silikon, Metallfasern, Polyamid oder andere Lösemittel beständigen Kunststoffen enthalten oder vollständig daraus bestehen. Ein offenporiger Schaum kann z.B. Glas, Silikon, Metall. Polyurethan oder andere Lösemittel beständigen Kunststoffen enthalten oder vollständig daraus bestehen.

Das Volumen des Vorratsbehälters beträgt bevorzugt 1-30ml, z.B. 2 ml, 2,5 ml, 5 ml, 6ml, 7,5 ml, 8ml, 10 ml, 12,5 ml, 15 ml, 20 ml, 22,5 ml oder 25 ml, kann jedoch auch jeden dazwischen liegenden Wert annehmen. Das Volumen des Vorratsbehälters kann jedoch bei geeigneter Indikation auch größer als 30 ml sein. Größere Volumina können z.B. 35, 40, 45 oder 50 ml betragen. Weiter bevorzugt ist der Vorratsbehälter durch eine manuell bedienbare Dosiervorrichtung verschlossen, an deren Ende sich wiederum die beschriebene Öffnung zum Aufsetzen auf die Haut bzw. die Applikationsöffnung befindet.

In einer anderen bevorzugten Ausführungsform ist die Dosiervorrichtung des Hautapplikators manuell betätigbar. Das heißt, dass die Dosiervorrichtung durch Ausübung von Druck auf ein dafür vorgesehenes Teil, z.B. den Applikatoraufsatz, betätigt werden kann und so eine vorherbestimmbare bzw. vorherbestimmte Dosis der leichtflüchtigen Flüssigkeit in die Applikatorkammer abgibt.

In einer bevorzugten Ausführungsform ist der Vorratsbehälter durch eine manuell bedienbare Dosiervorrichtung verschlossen, an deren Ende sich der Aufsatz mit der Oberkante zum Aufsetzen auf die Haut befindet. Bevorzugte Ausgestaltungen des Hautapplikators bzw. der Vorrichtung finden sich in den Figuren.

Die Dosiervorrichtung kann in einer bevorzugten Ausführungsform durch ein Aufdrücken auf die Haut betätigbar sein.

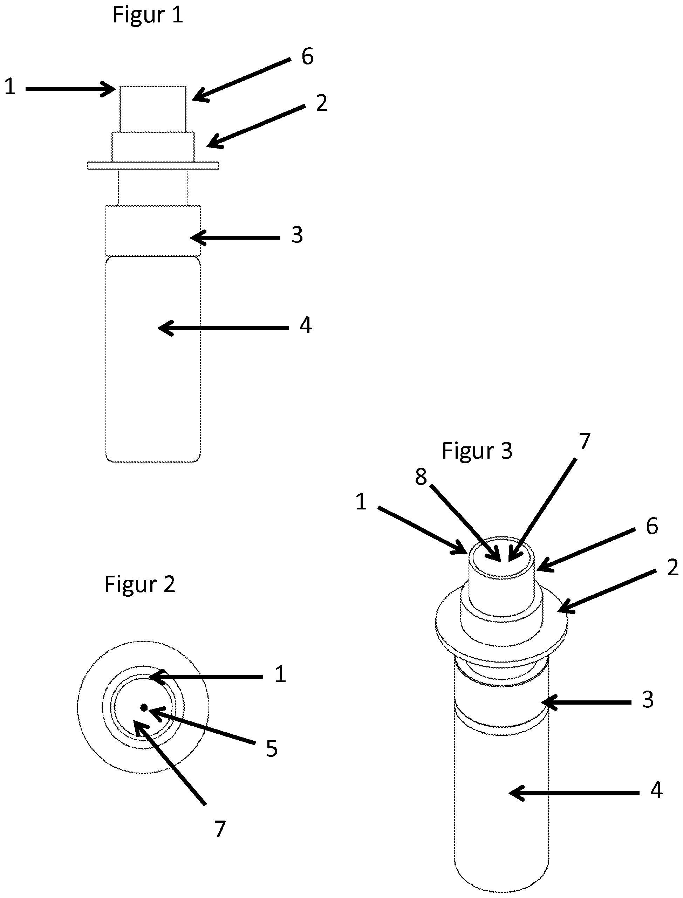

Die Einheit von Dosiervorrichtung und Aufsatz kann in einem Winkel von 180° auf dem Vorratsbehälter oder einem eventuell zwischen Vorratsbehälter und Dosiervorrichtung angebrachten Verbindungsstück aufliegen (siehe Figur 1), jedoch auch in jeden anderen Winkel zwischen 30 und 180° zum Vorratsbehälter oder dem Verbindungsstück annehmen, z.B. einen Winkel von 45°, 90° oder 120°.

In einer bevorzugten Ausführungsform ist die umlaufende Kante als eine bevorzugt an der Austrittsfläche umlaufende Dichtkante zum Aufsetzen auf die Haut ausgebildet.

Der Vorratsbehälter ist in einer weiteren bevorzugten Ausführungsform mit einem Treibgas druckbeaufschlagt. Das heißt, dass der Vorratsbehälter in dieser Ausführungsform zusätzlich zur leichtflüchtigen Flüssigkeit ein Treibgas enthält oder mit einem entsprechenden, das Treibgas enthaltenden Behälter gekoppelt ist und somit unter Druck steht. In dieser und anderen Ausführungsformen muss der Hautapplikator bzw. die Vorrichtung nur durch einen (leichten) Druck auf die Dosiervorrichtung betätigt werden, wodurch ein Ventil geöffnet wird, welches die leichtflüchtige Flüssigkeit oder Substanz in der gewünschten Menge in die Applikationskammer freigibt. In anderen Worten ist die Dosiervorrichtung derartig ausgebildet, dass die leichtflüchtige Flüssigkeit oder Substanz in die Applikationskammer freigegeben werden kann.

In einer anderen bevorzugten Ausführungsform befindet sich die Substanz in einer Menge von 1-30ml zusammen mit einem Treibgas in einem unter Druck stehenden Vorratsbehälter, der z.B. aus Glas, Kunststoff, elastischem Silikon oder einem Metall bestehen bzw. diese Materialien enthalten kann. Ein Teil des Volumens des Behälters (üblicherweise über 50% des Gesamtvolumens) wird dabei vom Wirkstoff eingenommen, z.B. Ether oder eine Kombination, und der anderer Teil besteht aus komprimiertem Treibmittel, welches teilweise flüssig vorliegen kann. Das Treibmittel kann ein Alkan oder ein Alkangemisch, Kohlendioxid, Stickstoff oder ein Fluorkohlenwasserstoff wie 1,1,1,2-Tetrafluorethan sein. Dabei ist dieser Behälter durch eine manuell bedienbare Dosiervorrichtung verschlossen, an deren Ende die beschriebene Öffnung zum Aufsetzen auf die Haut befindet.

In einer anderen bevorzugten Ausführungsform besteht der Vorratsbehälter aus oder enthält Glas, einen Kunststoff oder Metall. Geeignete Metalle sind zum Beispiel Aluminium, Kupfer, Eisen oder Messing und ihre Legierungen. Grundsätzlich ist jedes Material, welches gegenüber den obengenannten leichtflüchtigen Flüssigkeiten oder Substanzen chemisch beständig und dicht ist, für den erfindungsgemäßen Hautapplikator bzw. die Vorrichtung geeignet. Weitere Beispiele umfassen ein- oder mehrkomponentige Elastomere (z. B. Polyoxymethylen), Copolymerisate und Duromere.

In einer weiteren bevorzugten Ausführungsform beträgt die von der umlaufenden Kante umschlossene Fläche 4 bis 250 mm², z.B. 10, 50, 100, 150 oder 200 mm² oder jeden dazwischen befindlichen Wert.

Der Flächeninhalt an der Oberkante des Aufsatzes kann auch größer als 250 mm² sein, wenn dies für bestimmte Indikationen oder Anwendungen geboten scheint, z.B. bei großflächigen Infektionen oder zur Prävention.

In einer bevorzugten Ausführungsform ist zwischen der Dosiervorrichtung und dem Applikatoraufsatz ein Zerstäuber angeordnet.

Der erfindungsgemäße Hautapplikator bzw. die Vorrichtung ist zur Verwendung bei der Desinfektion von Haut gedacht, zur Desinfektion von Haut bei Verdacht auf eine Herpesinfektion. Beispielhafte Viren aus der Gruppe der Herpesviren sind an anderer Stelle genannt. Auch eignet sich der Hautapplikator bzw. die Vorrichtung zur Verwendung in der Behandlung von mit einer Infektion mit Herpesviren assoziierten Symptomen oder in der Behandlung von Infektionen mit einem oder mehreren Viren aus der Herpes-Gruppe.

Der erfindungsgemäße Hautapplikator bzw. die Vorrichtung kann zur Applikation von leicht flüchtigen Substanzen mit einem Dampfdruck von größer als 100hPa bei 20C° zur Desinfektion von Haut zum Beispiel bei Verdacht auf Vorhandensein von Herpes Simplex Viren 1 bis 6 oder Varizella Zoster Virus verwendet werden. Weiterhin ist die Verwendung des erfindungsgemäßen Hautapplikators bzw. der erfindungsgemäßen Vorrichtung zur Applikation von Diethylether oder Gemischen mit Diethylether zur Desinfektion von Haut bei Verdacht auf Vorhandensein von Viren der Herpesgruppe vorgesehen.

Auch von der Beschreibung umfasst ist ein Verfahren zur Desinfektion von Hautarealen unter Verwendung des erfindungsgemäßen Hautapplikators bzw. der erfindungsgemäßen Vorrichtung, umfassend Aufdrücken auf die Haut bis zur Erreichung der gewünschten Interaktionszeit des in die Applikationskammer des Hautapplikators bzw. der Vorrichtung freigesetzten Stoffes mit dem kontaktierenden Hautareal.

Die Beschreibung offenbart weiterhin ein Verfahren zur Desinfektion von Hautarealen unter Verwendung eines oben beschriebenen erfindungsgemäßen Hautapplikators/ einer erfindungsgemäßen Vorrichtung, umfassend die Schritte (a) Aufdrücken der umlaufenden Kante auf das Hautareal (b) Betätigen der Dosiervorrichtung, und (c) Verweilen lassen des Hautapplikators bzw. der Vorrichtung in der aufgedrückten Position für eine vorbestimmte Interaktionszeit. Die für den oben beschriebene erfindungsgemäßen Hautapplikator bzw. die erfindungsgemäße Vorrichtung beschriebenen bevorzugten Ausführungsformen sind gleichermaßen auf das beschriebene Verfahren anzuwenden.

Das Betätigen der Dosiervorrichtung erfolgt mit einem Druck, der ausreicht, um eine vorbestimmte oder vorbestimmbare Dosis der leicht flüchtigen Flüssigkeit auf das Hautareal aufzubringen.

In einer bevorzugten Ausführungsform des erfindungsgemäßen Verfahrens wird die Flüssigkeit als Aerosol freigesetzt.

In einer weiteren bevorzugten Ausführungsform wird der Schritt (b), das heißt die Auslösung von weiteren Betätigungen der Dosiervorrichtung während des Aufdrückens auf das Hautareal, mehrmals durchgeführt, d. h. z.B. mindestens zweimal, dreimal oder viermal. Dies ist vor allem bei einer manuell zu betätigenden Pumpdosiervorrichtung der Fall. Dabei kann/können der/die zusätzliche(n) Auslösungen direkt nach Schritt (b), aber auch nach Schritt (c) stattfinden. Bei einer durch Treibgas freigesetzten Dosis an leichtflüchtiger Flüssigkeit bzw. Substanz wird bevorzugt die korrekte Dosis durch einmaliges Betätigen freigesetzt.

Die Interaktionszeit bzw. die Verweildauer der umlaufenden Kante der Vorrichtung bzw. des Hautapplikators auf der Haut kann z.B. zwischen 10 s und 10 min betragen, z.B. 1, 2, 3, 4, 5, 6, 7, 8, oder 9 min.

Zuletzt bezieht sich die Erfindung auf die Verwendung der oben beschriebenen erfindungsgemäßen Vorrichtung bzw. Hautapplikators zur Applikation von leicht flüchtigen Flüssigkeiten/Substanzen mit einem Dampfdruck von größer als 57hPa bei 20C° zur Desinfektion von Haut, bevorzugt bei Verdacht auf Vorhandensein von Viren der Herpesgruppe, weiter bevorzugt bei Verdacht auf Vorhandensein von Herpes Simplex Viren 1 bis 6 oder des Varizella Zoster Virus.

In einer bevorzugten Ausführungsform ist die leichtflüchtige Flüssigkeit Diethylether oder ein Gemisch mit Diethylether wie an anderer Stelle beschrieben.

Die vorliegende Erfindung wird nachfolgend unter Bezugnahme auf die beigefügten Figuren eingehend erläutert. Es zeigen:
- Figur 1: eine Seitenansicht einer ersten Ausführungsform eines Hautapplikators,
- Figur 2: eine Draufsicht auf die Ausführungsform aus Figur 1,
- Figur 3: eine perspektivische Darstellung der Ausführungsform aus den Figuren 1 und 2,
- Figur 4: eine Seitenansicht einer zweiten Ausführungsform eines Hautapplikators,
- Figur 5: eine Draufsicht auf die Ausführungsform aus Figur 4,
- Figur 6: eine dritte Ausführungsform eines Hautapplikators,
- Figur 7: eine Seitenansicht einer vierten Ausführungsform eines Hautapplikators,
- Figur 8: eine Draufsicht auf das Ausführungsbeispiel aus Figur 7,
- Figur 9: eine perspektivische Darstellung der Ausführungsform aus den Figuren 7 und 8.

Figur 1 zeigt eine erste Ausführungsform eines Hautapplikators in seitlicher Ansicht. Die Figuren 2 und 3 zeigen eines Draufsicht bzw. eine perspektivische Darstellung des Hautapplikators aus Figur 1.

Um unnötige Wiederholungen zu vermeiden, werden die Figuren 1 bis 3 nachfolgend gemeinsam behandelt.

Der Hautapplikator besteht im Wesentlichen aus einem Vorratsbehälter 4, mit dem eine Dosiervorrichtung 2 gekoppelt ist, die entweder eine Pumpdosiervorrichtung (welche das flüssige Medium durch manuellen Druck auf den Auslöser pumpt und dosiert) oder im Fall von Treibgasanwendung eine Dosiereinrichtung (dosiert ohne die Notwendigkeit des Förderns der Flüssigkeit durch Pumpen) ist. An der Dosiervorrichtung ist ein Applikatoraufsatz 6 mit einer Applikationskammer 7 angeordnet, der stirnseitig eine Applikationsöffnung 8, die von einer umlaufenden Dichtkante 1, die im vorliegenden Ausführungsbeispiel die Oberkante des Applikatoraufsatzes 6 bildet, begrenzt ist, aufweist. Im vorliegenden Ausführungsbeispiel ist die Dosiervorrichtung 2 über ein Verbindungsstück 3 mit dem Vorratsbehälter 4 verbunden. Wird beispielsweise ein Vorratsbehälter aus Glas verwendet, so kann das Verbindungsstück 3 ein Gewinde zum Aufschrauben der Dosiervorrichtung 2 oder sonstige Verbindungsmittel zum Verbinden der Dosiervorrichtung 2 mit dem Vorratsbehälter umfassen. Der Applikatoraufsatz 6 des Ausführungsbeispiels aus den Figuren 1 bis 3 ist im Wesentlichen zylindrisch ausgebildet, wobei der Zylinder an einem von dem Vorratsbehälter 4 wegweisenden Ende die Applikationsöffnung 8 aufweist. Die Applikationsöffnung 8 ist durch die umlaufende Kante 1 begrenzt. Die umlaufende Kante definiert damit auch eine Austrittsfläche, die der maximalen Größe des bei einer Applikation zu behandelnden Hautareals entspricht.

Die Dosiervorrichtung 2 übergreift den Applikatoraufsatz 6 außenseitig und ist mit einem umlaufenden Kragen, der zur manuellen Betätigung der Dosiervorrichtung 2 vorgesehen ist, versehen. Durch Ausüben einer Kraft auf diesen umlaufenden Kragen kann die Dosiervorrichtung 2 in Richtung des Vorratsbehälters 4 bewegt und somit ein Pumpvorgang ausgelöst werden.

Hierdurch wird ein Sprühstoß des in dem Vorratsbehälter 4 befindlichen Mediums in die Applikationskammer 7 abgegeben. Alternativ kann der Vorratsbehälter 4 druckbeaufschlagt sein und durch Betätigung des umlaufenden Kragens ein Sprühstoß ausgelöst werden.

Eine Düsenöffnung 5, die als Zerstäuber wirkt, ist ausgangsseitig an der Dosiervorrichtung 2 vorgesehen und bewirkt eine Dispersion des geförderten Mediums.

Durch Aufsetzen der Oberkante 1 auf Haut entsteht die Applikationskammer 7, die außenseitig durch den z.B. zylindrischen Applikatoraufsatz 6, in dem die Düsenöffnung 5 angeordnet ist. Der Aufsatz kann jede geeignete Form annehmen, z.B. auch elliptisch oder eckig sein. Eine Kammerwandung kann aus einem Kunststoff, Metall, Glas oder elastischem Silikon bestehen. Die so begrenzte Apoplikationskammer 7 weist nach Aufsetzen auf die Haut ein vordefiniertes Volumen auf, das geeignet gewählt ist, das geförderte und ggf. zerstäubte Volumen des zu applizierenden Mediums in Form einer leichtflüchtigen Flüssigkeit aufzunehmen und die Wirkung auf das zu behandelnden Hautareal zu gewährleisten. Ein geeignetes Volumen beträgt z.B. 0,5 bis 8 cm³.

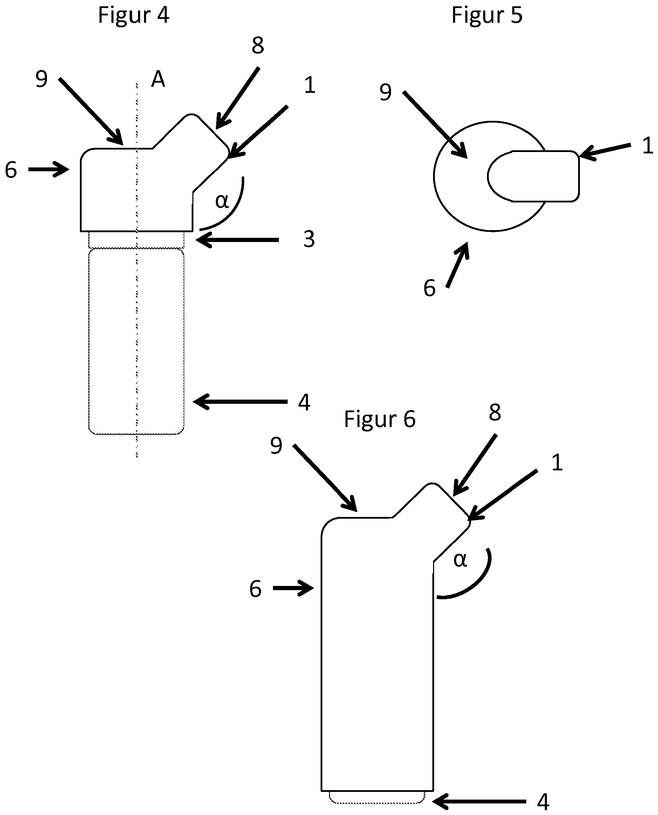

- Figur 4: zeigt eine seitliche Draufsicht eines zweiten Ausführungsbeispiels eines Hautapplikators, wobei der Applikatoraufsatz 6 samt umlaufender Kante 1 in einem Winkel kleiner als 180 ° zum Vorratsbehälter 4 angeordnet ist. In dieser Ausführungsform ist der Applikatoraufsatz 6 mit der Dosiervorrichtung 2 kombiniert und mit einer seitlich abgewinkelten Anformung ausgebildet, in der die Applikationsöffnung 8 angeordnet ist. Mit anderen Worten ist der Applikatoraufsatz derart ausgestaltet, dass die umlaufende Kante 1 als Teil eines in einem Winkel α < 180 ° zu einer durch den Vorratsbehälter 4 definierten Längsachse A des Aufsatzes 6 ausgebildet ist. Im vorliegenden Ausführungsbeispiel beträgt der Winkel α ca. 135°, doch es sind auch Ausgestaltungen mit einem Winkel von etwa 160° bis 60° möglich. Eine abgewinkelte Anordnung der Applikationskammer 7 in der Anformung ist vorteilhaft, da die Anwendung des Hautapplikators durch den Anwender z.B. durch bessere Sicht leichter kontrollierbar ist.
Durch die abgewinkelte Anformung an den Applikatoraufsatz 6 ergibt sich eine freie Oberfläche 9, auf die durch Fingerdruck gegenüber dem Behälterboden eine Dosierung ausgelöst werden kann. Somit fungiert die freie Oberfläche 9 in dieser Ausführungsform als Betätigungsfläche für die Dosiervorrichtung 2 (hier nicht dargestellt). Die umlaufende Kante 1 schließt nach Aufsetzen auf die Haut die Applikationskammer 7 ab, welche durch die abgewinkelte Anformung an denz.B. zylindrischen oder elliptischen Applikatoraufsatz 6 vor dem Zerstäuber 5 gebildet wird. Die Form der Anformung ist ähnlich variabel wie die des Applikatoraufsatzes 6 und kann z.B. rund oder elliptisch sein und insbesondere auch in ihrer Größe an die Anforderungen des jeweiligen Anwendungsfalls angepasst sein. Die Kammerwandung kann aus einem Kunststoff, Glas oder elastischem Silikon bestehen. Die Dosiersprüheinrichtung, z.B. integriert in den Verschluss des Behälters 4, z.B. ausgestaltet als Verbindungsstück 3, wird ausgelöst durch ein Drücken der Fläche 9 in Richtung des Bodens des Behälters 4. Die umlaufende Kante 1 kann insbesondere als Dichtkante ausgestaltet sein, die aus einem elastischen Material bestehen kann, das zur Abdichtung der Applikationskammer nach Aufsetzen auf die Haut geeignet ist.
- Figur 5: zeigt eine schematische Draufsicht auf den Hautapplikator aus Figur 4. In Figur 5 ist besonders deutlich die Betätigungsfläche 9 zu erkennen, auf die zur Auslösung der Dosiervorrichtung manuell Druck ausgeübt wird.
- Figur 6: zeigt eine seitliche Draufsicht einer dritten Ausführungsform eines Hautapplikators, bei der analog zu der Ausführungsform in Figur 4 die Applikationskammer 7 seitlich abgewinkelt angeordnet und in einer Anformung ausgebildet ist. Der Applikatoraufsatz ist in der vorliegenden Ausführungsform so ausgebildet, dass er den Vorratsbehälter 4 annähernd vollständig umschließt, wobei ein bodenseitiges Ende des Vorratsbehälters 4 aus dem Applikatoraufsatz 6 so weit hervorragt, dass ein Dosiervorgang ausgelöst werden kann. Dies geschieht im vorliegenden Beispiel durch Zusammendrücken der Betätigungsfläche 9 und des bodenseitigen Endes des Vorratsbehälters 4. Alternativ kann der Applikatoraufsatz 6 in Axialrichtung bis zum Boden des Vorratsbehälters 4 geführt sein oder diesen sogar überragen. Wenn der Behälterboden nicht mehr herausragt, sondern vom Applikatoraufsatz 6 überdeckt ist, wird eine unbeabsichtigte Auslösung, etwa in der Handtasche, vermieden, ohne dass eine beabsichtige Auslösung behindert wird. 9.

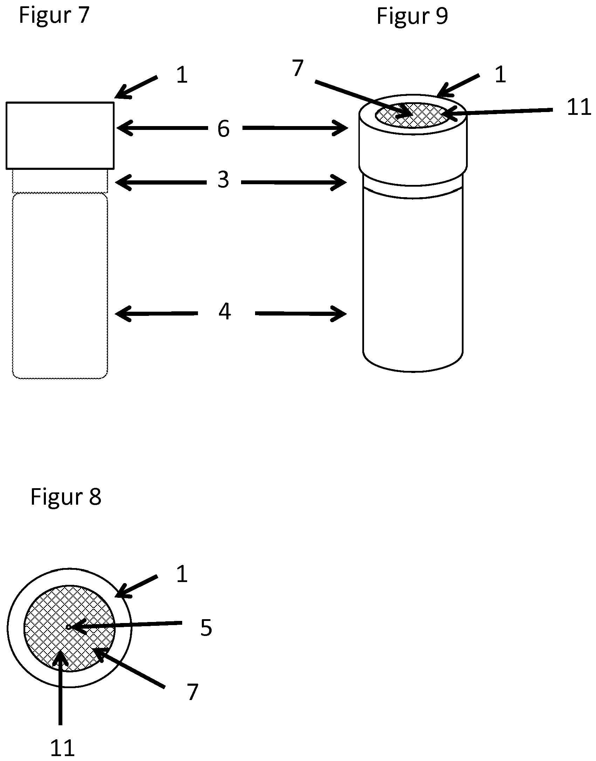

- Die Figuren 7 bis 9: zeigen in seitlicher Draufsicht (Figur 7), Draufsicht (Figur 8) und perspektivischer Darstellung (Figur 9) eine vierte Ausführungsform eines Hautapplikators, bei der die nötige Kompression zwischen Applikatoraufsatz 6 und Behälterboden zur Auslösung der (Pump)-Dosiervorrichtung durch Druck der umlaufenden Kante 1 gegen das zu behandelnde Hautareal erreicht wird. Auch hier schließt die umlaufende Kante 1 nach Aufsetzen auf die Haut eine Applikationskammer 7 ab, in der im vorliegenden Ausführungsbeispiel ein poröses Material, beispielsweise ein offenporiger Schaum, Watte oder ein Vlies angeordnet ist, um spontanes Ablaufen des Mediums bei Undichtigkeit einer Kammer-Haut Kontaktstelle zu vermeiden. Es sei an dieser Stelle erwähnt, dass das poröse Material 11 in sämtlichen Ausgestaltungen vorgesehen sein kann und der Pumpvorgang manuell oder durch Druckbeaufschlagung des Vorratsbehälters 4 ausgelöst werden kann.

## Patentansprüche

1. Hautapplikator für leichtflüchtige Flüssigkeiten mit einem Vorratsbehälter (4), einer mit dem Vorratsbehälter gekoppelte Dosiervorrichtung (2) zur Förderung eines vorbestimmbaren Flüssigkeitsvolumens, sowie einem Applikatoraufsatz (6) mit einer Applikationskammer (7), wobei die Applikationskammer einerseits mit der Dosiervorrichtung verbunden ist und andererseits eine von einer umlaufenden Kante (1) begrenzte Applikationsöffnung (8) zum Aufsetzen auf die Haut aufweist zur Verwendung in der Behandlung von Infektionen mit Viren aus der Herpes-Gruppe, **dadurch gekennzeichnet dass** der Vorratsbehälter (4) als leichtflüchtige Substanz Diethylether oder Chloroform oder Gemische mit Diethylether enthält und dass der Hautapplikator bis auf die Applikationsöffnung (8) im Wesentlichen geschlossen ist.

2. Hautapplikator nach Anspruch 1, **dadurch gekennzeichnet, dass** das Virus ausgewählt ist aus Herpes Simplex Viren 1 bis 6, bevorzugt Herpes Simplex 1 und 2, und Varizella Zoster Virus.

3. Hautapplikator nach einem der Ansprüche 1 und 2, **dadurch gekennzeichnet, dass** die Applikationskammer (7) nach Aufsetzen des Hautapplikator auf ein Hautareal ein Volumen von 0,1-8 cm³ bildet.

4. Hautapplikator nach einem der Ansprüche 1 bis 3 **dadurch gekennzeichnet, dass** in der Applikationskammer (7) ein gegenüber der leicht flüchtigen Flüssigkeit stabiles poröses Material (11), bevorzugt Watte, Vlies oder offenporiger Schaum, angeordnet ist.

5. Hautapplikator nach Anspruch 4, **dadurch gekennzeichnet, dass** die Dichte des porösen Materials (11) unter 0,5 g/cm³ beträgt.

6. Hautapplikator nach einem der Ansprüche 1 bis 5, **dadurch gekennzeichnet, dass** das Volumen des Vorratsbehälters (4) 1-30ml beträgt.

7. Hautapplikator nach einem der Ansprüche 1 bis 6, **dadurch gekennzeichnet, dass** der Vorratsbehälter (4) aus Glas oder einem Metall besteht.

8. Hautapplikator nach einem der Ansprüche 1 bis 7, **dadurch gekennzeichnet, dass** die Dosiervorrichtung (2) manuell betätigbar ist und bevorzugt eine Pumpdosiereinrichtung ist.

9. Hautapplikator nach einem der Ansprüche 1 bis 8, **dadurch gekennzeichnet, dass** die umlaufende Kante (1) als eine bevorzugt an einer Austrittsfläche (8) umlaufende Dichtkante zum Aufsetzen auf die Haut ausgebildet ist.

10. Hautapplikator nach einem der Ansprüche 1 bis 9, **dadurch gekennzeichnet, dass** der Vorratsbehälter (4) mit einem Treibgas druckbeaufschlagt ist.

11. Hautapplikator nach einem der Ansprüche 1 bis 10, **dadurch gekennzeichnet, dass** die von der umlaufenden Kante (1) umschlossene Fläche 4 bis 250 mm² beträgt.

12. Hautapplikator nach einem der Ansprüche 1 bis 11, **dadurch gekennzeichnet, dass** die Dosiervorrichtung (2) durch ein Aufdrücken auf die Haut betätigbar ist.

13. Hautapplikator nach einem der Ansprüche 1 bis 12, **dadurch gekennzeichnet, dass** zwischen der Dosiervorrichtung und dem Applikatoraufsatz (6) ein Zerstäuber angeordnet ist.

## Claims

1. Skin applicator for volatile liquids with a storage container (4), a dosing device (2) coupled to the storage container for conveying a predeterminable volume of liquid, as well as an applicator attachment (6) with an application chamber (7), wherein the application chamber is connected on the one hand to the dosing device and on the other hand has an application opening (8) confined by a circumferential edge (1) for placing on the skin for use in the treatment of infections with viruses from the herpes group, **characterized in that** the storage container (4) contains as volatile substance diethyl ether or chloroform or mixtures with diethyl ether and that the skin applicator is essentially closed except for the application opening (8).

2. Skin applicator according to claim 1, **characterized in that** the virus is selected from herpes simplex viruses 1 to 6, preferably herpes simplex 1 and 2, and varicella zoster virus.

3. Skin applicator according to any one of claims 1 and 2, **characterized in that** the application chamber (7) forms a volume of 0.1-8 cm³ after placing the skin applicator on a skin area.

4. Skin applicator according to any one of claims 1 to 3, **characterized in that** a porous material (11), preferably wadding, fleece or open-pored foam, which is stable with respect to the volatile liquid, is arranged in the application chamber (7).

5. Skin applicator according to claim 4, **characterized in that** the density of the porous material (11) is below 0.5 g / cm³.

6. Skin applicator according to any one of claims 1 to 5, **characterized in that** the volume of the storage container (4) is 1-30ml.

7. Skin applicator according to any one of claims 1 to 6, **characterized in that** the storage container (4) consists of glass or a metal.

8. Skin applicator according to any one of claims 1 to 7, **characterized in that** the dosing device (2) can be operated manually and is preferably a pump dosing device.

9. Skin applicator according to any one of claims 1 to 8, **characterized in that** the circumferential edge (1) is designed as a sealing edge preferably on an exit surface (8) for placing on the skin.

10. Skin applicator according to any one of claims 1 to 9, **characterized in that** the storage container (4) is pressurized with a propellant gas.

11. Skin applicator according to any one of claims 1 to 10, **characterized in that** the area enclosed by the circumferential edge (1) is 4 to 250 mm².

12. Skin applicator according to any one of claims 1 to 11, **characterized in that** the dosing device (2) can be actuated by pressing it onto the skin.

13. Skin applicator according to any one of claims 1 to 12, **characterized in that** an atomizer is arranged between the dosing device and the applicator attachment (6).

## Revendications

1. Applicateur cutané pour liquides volatils, comprenant un récipient de stockage (4), un dispositif de dosage (2) couplé au récipient de stockage et destiné à acheminer un volume de liquide prédéterminé, ainsi qu'une pièce rapportée d'applicateur (6) ayant une chambre d'application (7), ladite chambre d'application étant reliée d'une part au dispositif de dosage et comprenant d'autre part une ouverture d'application (8) délimitée par un bord circonférentiel (1) et destinée à être placée sur la peau pour l'utilisation dans le traitement d'infections par des virus du groupe de l'herpès, **caractérisé par le fait que** le récipient de stockage (4) contient en tant que substance volatile de l'éther diéthylique ou du chloroforme ou des mélanges comprenant de l'éther diéthylique, et que, à l'exception de l'ouverture d'application (8), l'applicateur cutané est pour l'essentiel fermé.

2. Applicateur cutané selon la revendication 1, **caractérisé par le fait que** le virus est choisi parmi les virus herpès simplex 1 à 6, de préférence herpès simplex 1 et 2, et le virus varicelle-zona.

3. Applicateur cutané selon la revendication 1 et 2, **caractérisé par le fait que** la chambre d'application (7) forme un volume compris entre 0,1 - 8 cm³ après que l'applicateur cutané a été placé sur une zone de peau.

4. Applicateur cutané selon l'une quelconque des revendications 1 à 3, **caractérisé par le fait qu'**un matériau poreux (11), de préférence de l'ouate, du non-tissé ou de la mousse à pores ouverts, qui est stable par rapport au liquide volatil, est disposé à l'intérieur de la chambre d'application (7).

5. Applicateur cutané selon la revendication 4, **caractérisé par le fait que** la densité du matériau poreux (11) est inférieure à 0,5 g/cm³.

6. Applicateur cutané selon l'une quelconque des revendications 1 à 5, **caractérisé par le fait que** le volume du récipient de stockage (4) est compris entre 1 et 30 ml.

7. Applicateur cutané selon l'une quelconque des revendications 1 à 6, **caractérisé par le fait que** le récipient de stockage (4) est constitué de verre ou d'un métal.

8. Applicateur cutané selon l'une quelconque des revendications 1 à 7, **caractérisé par le fait que** le dispositif de dosage (2) peut être actionné manuellement et est de préférence un dispositif de dosage à pompe.

9. Applicateur cutané selon l'une quelconque des revendications 1 à 8, **caractérisé par le fait que** le bord circonférentiel (1) est conçu comme un bord d'étanchéité qui, de préférence, s'étend autour d'une surface de sortie (8) et est destiné à être posé sur la peau.

10. Applicateur cutané selon l'une quelconque des revendications 1 à 9, **caractérisé par le fait que** le récipient de stockage (4) est pressurisé avec un gaz propulseur.

11. Applicateur cutané selon l'une quelconque des revendications 1 à 10, **caractérisé par le fait que** la surface délimitée par le bord circonférentiel (1) est de 4 à 250 mm².

12. Applicateur cutané selon l'une quelconque des revendications 1 à 11, **caractérisé par le fait que** le dispositif de dosage (2) peut être actionné en étant plaqué contre la peau.

13. Applicateur cutané selon l'une quelconque des revendications 1 à 12, **caractérisé par le fait qu'**un atomiseur est disposé entre le dispositif de dosage et la pièce rapportée d'applicateur (8).
